# EUROPEAN PATENT APPLICATION

(11) **EP 3 795 185 A1**
(43) Date of publication of application: **24.03.2021**
(21) Application number: 19198938.3
(22) Date of filing: 23.09.2019
(51) Int. Cl.: A61L 27/22, A61F 2/46

(54) **BONE GRAFT MATERIAL FOR USE IN A SPINAL FUSION METHOD**

(71) Applicant: Kuros Biosurgery AG, 8952 Schlieren (CH)
(72) Inventor: VOGT, Lorenz, 8344 Baeretswil (CH); SAUDAN, Philippe, 8422 Pfungen (CH); IRVINE, Alistair Simpson, 8915 Hausen am Albis (CH)
(74) Representative: Hepp Wenger Ryffel AG

(57) **Abstract**

The invention concerns a bone graft material for use in a spinal fusion method, wherein the material comprises i) a composition for forming a matrix, comprising at least a first matrix material precursor component, and a second matrix material precursor component, capable of forming a matrix by crosslinking of the precursor components under appropriate conditions; and ii) a bioactive factor, which is biologically active to stimulate bone formation between two vertebrae, and for effecting or supporting spinal fusion; wherein the spinal fusion method comprises the steps of applying a cage in between the two vertebrae, which cage is not pre-filled with the bone graft material; and subsequently applying the bone graft material adjacent to the cage and/or into the cage, such that essentially the entire remaining volume between the two vertebrae is filled with the bone graft material. The invention thus allows for ease of use while at the same time resulting in a matrix that is more homogeneous.

## Description

The present invention relates to the field of spinal fusion and, more specifically, to a certain bone graft material for use in a specific spinal fusion method as well as a related kit-of-parts.

Intervertebral discs can degenerate or otherwise become damaged over time. In some instances, an intervertebral implant (frequently referred to as a 'cage', but also commonly referred to as a spacer) can be positioned within the space previously occupied by a disc. These implants are to maintain the desired spacing between adjacent vertebrae.

Bone graft materials are frequently used together with spinal implants, both inside and adjacent to / surrounding the cage. This typically facilitates the fusion of adjacent vertebrae.

Various bone graft materials are currently in use. First, there are autologous bone graft, also referred to as autografts, which are the gold-standard of spinal fusion. However, harvesting is not possible for all patients; in any event, the procedure causes pain at the harvest site, comes along with a risk of secondary infections and is not ideal from a handling perspective. Second, rhBMP-2 based procedures are highly efficient; however, the burst release is disadvantageous, it does not affect cells that are not yet committed to form bone, inflammation and swelling is common and ectopic bone formation may occur. Other generally less efficacious bone grafts may also be used such as allografts, demineralised bone matrix, stem cell-based grafts or synthetic grafts,

Yet a further approach is known from WO 2012/123038 A1 which discloses the use of a specific bone graft material as a bone graft material in conjunction with a cage, i.e. a fibrin matrix that is supplemented with PTH. As disclosed therein, the cage is pre-filled with bone graft material, and the bone graft material is allowed to initially polymerize before it is inserted between two vertebrae. Yet further bone graft material is added adjacent to the cage in order to essentially completely fill the remaining volume between the two vertebrae.

This procedure on the one hand allows for a reliable filling of the cage as such, since the filling of the cage may still be controlled before the cage is inserted. On the other hand, homogeneity of the overall fibrin matrix may be imperfect, since the polymerization of the material within the cage and outside the cage is not proceeding in a synchronized manner and at a given point in time during polymerization, the material inside the cage is more completely polymerized than the surrounding material. Moreover, the use of multiple kits for the preparation of the bone graft material is necessary, i.e. a first one for the pre-filling of the cage; and, subsequently, at least one further kit for the addition of yet further bone graft material adjacent to the cage after insertion. This procedure may be perceived cumbersome by practitioners and may be inefficient in that two kits are used rather than one.

It is thus an objective of the present invention to overcome the above-mentioned drawbacks, and in particular to provide a bone graft material and a related method of application that allow for ease of use while at the same time resulting in a matrix that is more homogeneous.

These objectives are fulfilled by the subject-matter of the independent claims. Preferred embodiments are outlined in the respective dependent claims.

A first aspect of the invention relates to a bone graft material for use in a spinal fusion method, in particular for lumbar spine and/or thoracic spine, wherein the material comprises
i) a composition for forming a matrix, comprising at least a first matrix material precursor component, and a second matrix material precursor component, capable of forming a matrix by crosslinking of the precursor components under appropriate conditions; and
ii) a bioactive factor, which is biologically active to stimulate bone formation between two vertebrae, and for effecting or supporting spinal fusion, the bioactive factor being suitable to be releasably incorporated in the matrix upon crosslinking of the matrix material precursor components;
wherein the spinal fusion method comprises the steps of
- applying a cage in between the two vertebrae, which cage is not pre-filled with the bone graft material; and
- subsequently applying the bone graft material adjacent to the cage and/or into the cage, such that essentially the entire remaining volume between the two vertebrae is filled with the bone graft material.

A suitable bone graft material as such, as defined in items i) and ii), above, is known as such e.g. from WO 2012/123038 A1; the disclosure thereof is incorporated herein by reference with respect to the compositions of the bone graft materials disclosed therein.

It has surprisingly been found that, contrary to the common earlier belief that a cage must be pre-filled with the bone graft material in order to achieve an acceptable result, the bone graft material may well be applied only after the cage has been inserted in between the two vertebrae without having been pre-filled. Contrary to initial expectations, the bone graft material, in particular the one disclosed in WO 2012/123038 A1, may reliably flow around and into the cage to almost perfectly fill the remaining volume between the two vertebrae. This greatly facilitates the overall procedure since no bone graft material needs to be placed inside the cage before it is inserted, and there is no need to await an initial stage of polymerization of the material in the cage before insertion. Further, since all bone graft material is inserted into the space between the vertebrae at essentially the same time and at the same polymerization stage, the overall homogeneity of the matrix is improved.

According to a preferred embodiment, the bioactive factor is PTH or a biologically active fragment thereof, preferably PTH₁₋₃₄; or a peptide comprising PTH or a biologically active fragment thereof, preferably PTH₁₋₃₄. Any such suitable bioactive factors are disclosed in WO 2012/123038 A1; the disclosure thereof is incorporated herein by reference with respect to bioactive factors of the bone graft materials disclosed therein.

In further preferred embodiments, the peptide is a fusion peptide comprising at least two domains, and wherein the first domain comprises PTH or a biologically active fragment thereof, preferably PTH₁₋₃₄, and the second domain comprises a crosslinkable substrate domain. Again, this is described in any detail in WO 2012/123038 A1; the disclosure thereof is incorporated herein by reference with respect to the suitable crosslinkable substrate domains.

Preferably, as outlined in WO 2012/123038 A1 and as incorporated by reference herein, the crosslinkable substrate domain is or comprises a transglutaminase substrate domain, preferably a factor XIIIa substrate domain.

In further preferred embodiments, the fusion peptide of the bone graft material comprises an enzymatic or hydrolytic degradation site between the first and the second domain. The specific disclosure of WO 2012/123038 A1 with respect to suitable enzymatic or hydrolytic degradation sites is incorporated by reference herein.

In further preferred embodiments, the matrix is fibrin; and the composition for forming the matrix comprises fibrinogen, thrombin and a calcium source. The specific disclosure in WO 2012/123038 relating to this composition, these constituents thereof and their respective relation is incorporated herein by reference.

A further aspect of the present invention pertains to a kit-of-parts for use in a spinal fusion method, in particular for lumbar spine and/or thoracic spine, comprising
- a bone graft material as outlined above;
- a technical document as instructions for use of the bone graft material in a spinal fusion method as set forth below;
- optionally, a syringe for containing the bone graft material before it is applied in between the two vertebrae;
- further optionally, a cannula to be used with the syringe, for delivery of the bone graft material in between the two vertebrae, which cannula has
   - a length of at least 40mm, preferably at least 60mm, further preferably at least 100mm;
   - an inner diameter of at least 0.51mm (21G), preferably in the range of 0.51mm (21G) to 3.81mm (7G), further preferably in the range of 0.84mm (18G) to 2.69mm (10G), further preferably in the range of 1.19mm (16G) to 1.6mm (14G).

The technical document as instructions for use may be contained in the kit-of-parts e.g. by way of an instruction leaflet, or by way of a link to an instruction for use accessible over the internet. In any event, the instructions for use and the bone graft material specifically refer to each other.

It has been found that the aforementioned length of the cannula facilitates the application of the bone graft material in between to vertebrae in vivo, when no pre-filled cage is being used.

It has further been found that the aforementioned inner diameter of the cannula allow for efficient application of the bone graft material in between to vertebrae in vivo, when no pre-filled cage is being used, in one go and without the use of multiple kits of bone graft material being necessary.

The kit-of parts may further comprise a cage that is suitable for applying in between two vertebrae.

Contrary to the previously known approach to insert a pre-filled cage in between the vertebrae, the bone graft material is inserted in the space between the vertebrae in one go, only after the (empty) cage has already been inserted. This can most easily be achieved by a cannula with the aforementioned length which allows to reliably reach as much into the space between the vertebrae of a human as necessary in order to essentially fill the whole space between the vertebrae. The aforementioned inner diameter of the cannula allows for a reasonable flow rate to be achieved when the material is applied with the help of a manually operated syringe, as is typically the case.

In preferred embodiments, the kit-of-parts further comprises a technical leaflet with instructions for use of the bone graft material and the cage in a spinal fusion method as set forth below.

Accordingly, yet a further aspect of the invention pertains to a spinal fusion method, in particular for lumbar spine and/or thoracic spine, comprising the steps of
i) providing a cage that is suitable for applying in between two vertebrae;
ii) providing a bone graft material for use in a spinal fusion method, wherein the material comprises
   a. a composition for forming a matrix, comprising at least a first matrix material precursor component, and a second matrix material precursor component, capable of forming a matrix by crosslinking of the precursor components under appropriate conditions; and
   b. a bioactive factor, which is biologically active to stimulate bone formation between two vertebrae, and for effecting or supporting spinal fusion, the bioactive factor being suitable to be releasably incorporated in the matrix upon crosslinking of the matrix material precursor components;
iii) applying the cage in between the two vertebrae, which cage is not pre-filled with the bone graft material; and
iv) subsequently applying the bone graft material adjacent to the cage and/or into the cage, such that essentially the entire remaining volume between the two vertebrae is filled with the bone graft material.

In preferred embodiments, the bone graft material is applied in step iv) when between 30 seconds and 420 seconds, preferably between 60 seconds and 240 seconds, have lapsed after the first and the second matrix material precursor components have been first brought in contact with each other. In these time frames, it has been found that the material particularly reliably flows around and fills essentially the whole space between the two vertebrae.

It is further preferred that step iv) is carried out in a time frame of between 5 seconds and 60 seconds. It has been found that it generally improves homogeneity of the resulting matrix when the application itself is concluded in no more than 60 seconds.

In further preferred embodiments, the first and the second matrix material precursor components are mixed for about 20 seconds to about 40 seconds, preferably for about 30 seconds after having been first brought in contact with each other. It has been found that such mixing times are on the one hand sufficient in order to reliably allow for a homogeneous mixing, while at the same time the inevitable initial polymerization does not yet proceed to an extent that would hamper the subsequent application of the material.

In yet further preferred embodiments, the bone graft material is allowed to undergo initial polymerization after mixing and before step iv) for about 20 seconds to about 40 seconds, preferably for about 30 seconds. Such initial polymerization before application has been found to improve the handling properties of the material upon application, while at the same time not hampering the homogeneous formation of the matrix.

In yet further preferred embodiments, step iv) is carried out before the polymerization of the bone graft material has reached the gel point; preferably at least 0.5 minute, preferably 1.0 minute, further preferably 1.5 minutes, more preferably at least 3 minutes before the graft material has reached the gel point.

The gel point is determined as described in Chernysh et al. in Blood 2011 Apr 28; 117(17): 4609-4614.

As outlined above, the composition for forming a matrix comprises at least a first matrix material precursor component and a second matrix material precursor component, which two components are capable of forming a matrix by crosslinking of the precursor components under appropriate conditions. However, the skilled person will readily appreciate that the general concept of the present invention is even more generally applicable by using any kind of matrix material with characteristics that allow for a change of its rheologic properties from a flowable state (before and during application *in vivo*) to a non-flowable state (after application *in vivo*). As understood herein, the term 'flowable' means that a composition is ejectable from a syringe with an opening of 3mm diameter by manual operation; and the term 'non-flowable' means that a composition is not ejectable from a syringe with an opening of 3mm diameter by manual operation. This change in rheologic properties may be occasioned by enzymatic action, chemically, or by change of temperature. Any injectable bone graft substitute material can be used, such as those disclosed in Injury (2011) Sep; 42 Suppl 2: S30-4. For instance, biodegradable polymers (cross-linked and non-crosslinked) may be used in the context of the present invention as outlined above; as well as cements. Specifically, biodegradable polymers for use in the context of the present invention are e.g. those described in WO 2006/067221 (hydrogels); and Polymers (Basel) 2011 Sep 1; 3(3): 1377-1397 (PLGA). Cements for use in the context of the present invention are e.g. calcium phosphate / calcium sulphate based cements, such as e.g. PRO-DENSE™ (Wright Medical Group).

The invention will be described in the following by way of a specific embodiment which is not intended to limit the overall concept of the invention as discussed above in any way.

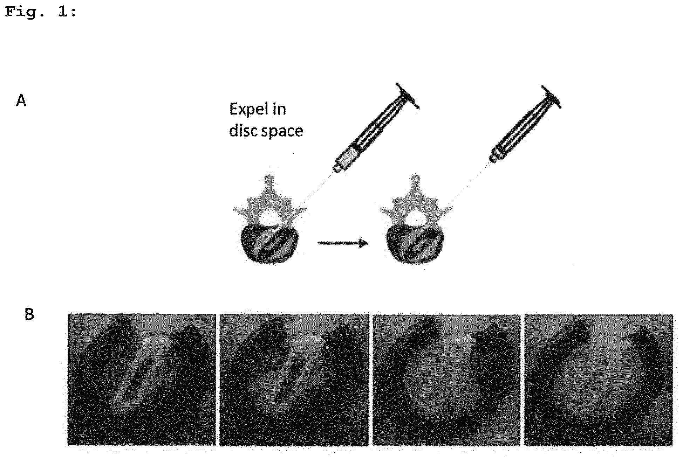

- Fig. 1A:: Schematic illustration of the application of the bone graft material into and next to the previously inserted cage;
- Fig. 1B:: Experimental testing of application of the bone graft material using a disc place model; the individual pictures show the bone graft flow at different time points.

### Preparation of bone graft material

The bone graft material consists of a TGplPTH₁₋₃₄ peptide solution and a fibrin sealant, as previously disclosed in WO 2012/123038 A1. This bone graft material is prepared on-site immediately prior to use as follows.

In the non-sterile field:
1. Thaw the vial of TGplPTH₁₋₃₄ solution at ambient conditions.

In the sterile field:
2. Thaw and warm the ARTISS pre-filled 10 ml syringe according to the preparation instructions described in the prescribing information from Baxter for ARTISS [Fibrin Sealant (Human)].
3. Using aseptic transfer technique and a 1 ml syringe, withdraw 0.5 mL of the TGplPTH₁₋₃₄ solution (see 1 above), holding up the inverted vial to facilitate withdrawal.
4. Transfer the complete 0.5 ml volume of TGplPTH₁₋₃₄ solution from the 1 ml syringe to a 10 ml syringe, using a female luer to female luer connector.

After complete thawing and warming of the ARTISS pre-filled 10 ml syringe, remove the cap and connect the nozzles of the double-chamber syringe to the joining piece, ensuring that they are firmly fixed. Secure the joining piece by fastening the tether strap to the double chamber syringe and insert the double plunger.
5. Using the female luer to female luer connector and connect the ARTISS double chamber syringe with the 10 mL syringe containing the 0.5 mL TGplPTH₁₋₃₄ solution.

### Immediately prior to application of the bone graft material:

6. Expel the content from the ARTISS syringe into the 10 mL syringe containing 0.5mL of the TGplPTH₁₋₃₄ solution. Remove the ARTISS double chamber syringe and joining piece from the luer connector and connect an empty 10 mL syringe to the 10 mL syringe containing TGplPTH₁₋₃₄ and ARTISS.

### (Step 6 is equal to the start of mixing the constituent parts.)

7. Mix the TGplPTH₁₋₃₄ solution and the ARTISS by transferring the mixture back and forth 5 times between the two syringes.
8. Remove the empty syringe and the luer connector and connect the syringe containing the bone graft material (TGplPTH₁₋₃₄ fibrin sealant) with a cannula (i.e. 14G).

### (Steps 6-8, i.e. the combining and mixing of the TGplPTH₁₋₃₄ solution and ARTISS lasts approx. 30 seconds.)

9. Incubate the KUR-113 Bone Graft for at least 30 seconds, to allow initial polymerization of the fibrin gel into a moldable fibrin matrix.
10. Immediately before application, expel and discard the first several drops from the application cannula.

### Application of Bone Graft in disc space Modell:

The disc space model allows to simulate the application of the bone graft material as an adjunctive therapy to hardware in spinal fusion procedures. The model contains a space comparable to the shape and size of cleared disc space and an aperture at the top for the insertion of a spinal implant ('cage') and the application of the bone graft material; see Fig. 1B. To monitor the procedure and visually inspect the product, the disc space model was made with a transparent plexiglass pane.

The application of the bone graft material after an empty spinal implant had been placed in the cleared disc space was studied using the disc space model. Briefly, an empty spinal implant was inserted in the disc space model. Then the aforementioned bone graft material was prepared as described above and injected in the disc space model using a 14 G cannula that was placed adjacent to the inserted spinal implant. As shown in the time lapse images of Figure 1B, although the cannula was placed next to the inserted spinal implant, upon expelling of the bone graft material, the disc space and the cage are homogenously filled with bone graft. Hence, surprisingly application of the bone graft material adjacent to an inserted spinal implant allows to homogenously fill the spinal implant and disc space with a single application after a spinal implant has already been inserted in the disc space.

Similar results have been obtained when bone graft material was inserted into the spinal implant instead of the aforementioned procedure (not shown in detail), such that the space between the two vertebrae is filled with the bone graft material flowing from inside of the spinal implant to the outer, remaining space.

## Claims

1. A bone graft material for use in a spinal fusion method, wherein the material comprises
i) a composition for forming a matrix, comprising at least a first matrix material precursor component, and a second matrix material precursor component, capable of forming a matrix by crosslinking of the precursor components under appropriate conditions; and
ii) a bioactive factor, which is biologically active to stimulate bone formation between two vertebrae, and for effecting or supporting spinal fusion;
wherein the spinal fusion method comprises the steps of
- applying a cage in between the two vertebrae, which cage is not pre-filled with the bone graft material; and
- subsequently applying the bone graft material adjacent to the cage and/or into the cage, such that essentially the entire remaining volume between the two vertebrae is filled with the bone graft material.

2. A bone graft material of claim 1, wherein the bioactive factor is suitable to be releasably incorporated in the matrix upon crosslinking of the matrix material precursor components.

3. A bone graft material of any of claims 1 or 2, wherein the bioactive factor is PTH or a biologically active fragment thereof, preferably PTH₁₋₃₄; or a peptide comprising PTH or a biologically active fragment thereof, preferably PTH₁₋₃₄.

4. A bone graft material of claim 3, wherein the peptide is a fusion peptide comprising at least two domains, and wherein the first domain comprises PTH or a biologically active fragment thereof, preferably PTH₁₋₃₄, and the second domain comprises a crosslinkable substrate domain.

5. A bone graft material of claim 4, wherein the crosslinkable substrate domain is or comprises a transglutaminase substrate domain, preferably a factor XIIIa substrate domain.

6. A bone graft material of any of claims 4 or 5, wherein the fusion peptide comprises an enzymatic or hydrolytic degradation site between the first and the second domain.

7. A bone graft material of any of the preceding claims,
wherein the matrix is fibrin; and the composition for formig the matrix comprises fibrinogen, thrombin and a calcium source.

8. A kit-of-parts for use in a spinal fusion method, comprising
- a bone graft material of any of the preceding claims;
- a technical document as instructions for use of the bone graft material in a spinal fusion method as set forth in any of claims 10 to 15;
- optionally, a syringe for containing the bone graft material before it is applied in between the two vertebrae;
- further optionally, a cannula to be used with the syringe, for delivery of the bone graft material in between the two vertebrae, which cannula has
- a length of at least 40mm, preferably at least 60mm, further preferably at least 80mm;
- an inner diameter of at least 0.51mm (21G), preferably in the range of 0.51mm (21G) to 3.81mm (7G), further preferably in the range of 0.84mm (18G) to 2.69mm (10G), further preferably in the range of 1.19mm (16G) to 1.6mm (14G) .

9. A kit of parts of claim 8, further comprising a cage that is suitable for applying in between two vertebrae.

10. A spinal fusion method, comprising the steps of
i) providing a cage that is suitable for applying in between two vertebrae;
ii) providing a bone graft material for use in a spinal fusion method, wherein the material comprises
a. a composition for forming a matrix, comprising at least a first matrix material precursor component, and a second matrix material precursor component, capable of forming a matrix by crosslinking of the precursor components under appropriate conditions; and
b. a bioactive factor, which is biologically active to stimulate bone formation between two vertebrae, and for effecting or supporting spinal fusion, the bioactive factor in particular being suitable to be releasably incorporated in the matrix upon crosslinking of the matrix material precursor components;
iii) applying the cage in between the two vertebrae, which cage is not pre-filled with the bone graft material; and
iv) subsequently applying the bone graft material adjacent to the cage and/or into the cage, such that essentially the entire remaining volume between the two vertebrae is filled with the bone graft material.

11. A spinal fusion method of claim 10, wherein the bone graft material is applied in step iv) when between 30 seconds and 420 seconds, preferably between 60 seconds and 240 seconds, have lapsed after the first and the second matrix material precursor components have been first brought in contact with each other.

12. A spinal fusion method of any of claims 9 or 10, wherein step iv) is carried out in a time frame of between 5 seconds and 60 seconds.

13. A spinal fusion method of any of claims 9 to 11, wherein the first and the second matrix material precursor components are mixed for about 20 seconds to about 40 seconds, preferably for about 30 seconds after having been first brought in contact with each other.

14. A spinal fusion method of claim 12, wherein the bone graft material is allowed to undergo initial polymerization after mixing and before step iv) for about 20 seconds to about 40 seconds, preferably for about 30 seconds.

15. A spinal fusion method of any of claims 9 to 13, wherein step iv) is carried out before the polymerization of the bone graft material has reached the gel point, preferably at least 0.5 minute, preferably 1.0 minute, further preferably 1.5 minutes, more preferably at least 3 minutes before the graft material has reached the gel point.
